# FASCICULE DE BREVET EUROPEEN

(11) **EP 1 772 108 B1**
(45) Date de publication et mention de la délivrance du brevet: **11.11.2015**
(21) Numéro de dépôt: 06290829.8
(22) Date de dépôt: 23.05.2006
(51) Int. Cl.: A61B 17/68, A61F 2/28

(54) **Cale destinée à l'ostéotomie tibiale ou fémorale**
Keil für tibiale oder femorale Osteotomie
Wedge for tibial or femoral osteotomy

(30) Priorité: 30.06.2005 FR 0506727
(43) Date de publication de la demande: 11.04.2007
(73) Titulaire: Kasios, 31140 Launaguet (FR)
(72) Inventeur: Hernigou, Philippe, 75015 Paris (FR); Lerch, Alain, 31600 Muret (FR); Guena, Nicolas, 75016 Paris (FR)
(74) Mandataire: Eidelsberg, Olivier Nathan

(56) Documents cités:
- EP-A- 0 574 098
- WO-A-00/74608
- WO-A-98/52498
- WO-A-99/52473
- WO-A-02/096324
- DE-A- 2 910 627
- DE-C- 19 610 715
- DE-U- 20 010 346
- GB-A- 2 093 701
- US-A- 5 766 251
- US-A1- 2001 039 455
- US-A1- 2003 105 526
- US-B1- 6 283 997

## Description

La présente invention se rapporte à une cale destinée à l'ostéotomie tibiale ou fémorale.

Pour des opérations du type genu valgum ou genu varum après avoir pratiqué dans le tibia ou le fémur une ostéotomie, on insère dans cette ostéotomie une cale dont une extrémité est enserrée entre les bords de l'ostéotomie pratiquée.

On utilise de préférence des cales réalisées en un matériau, d'une part, biocompatible et, d'autre part, permettant la colonisation du tissu spongieux, le matériau étant notamment en céramique.

De DE 29 106 627 on connaît un implant formé de fils en céramique qui se croisent mutuellement en un réseau. Cet implant a une bonne porosité mais n'est pas suffisamment résistant au risque d'affaissement au niveau du périoste.

De DE WO 98/52 498, on connaît un implant pour le remplacement d'os ou de cartilage. Il ne s'agit pas d'une cale destinée à l'ostéotomie tibiale ou fémorale. L'implant est constitué de barreaux en céramique qui se croisent mutuellement en un réseau. Cet implant est poreux mais ne serait pas suffisamment résistant au risque d'affaissement au niveau du périoste si on l'utilisait comme cale pour une ostéotomie tibiale ou fémorale, ce à quoi il n'est d'ailleurs pas destiné.

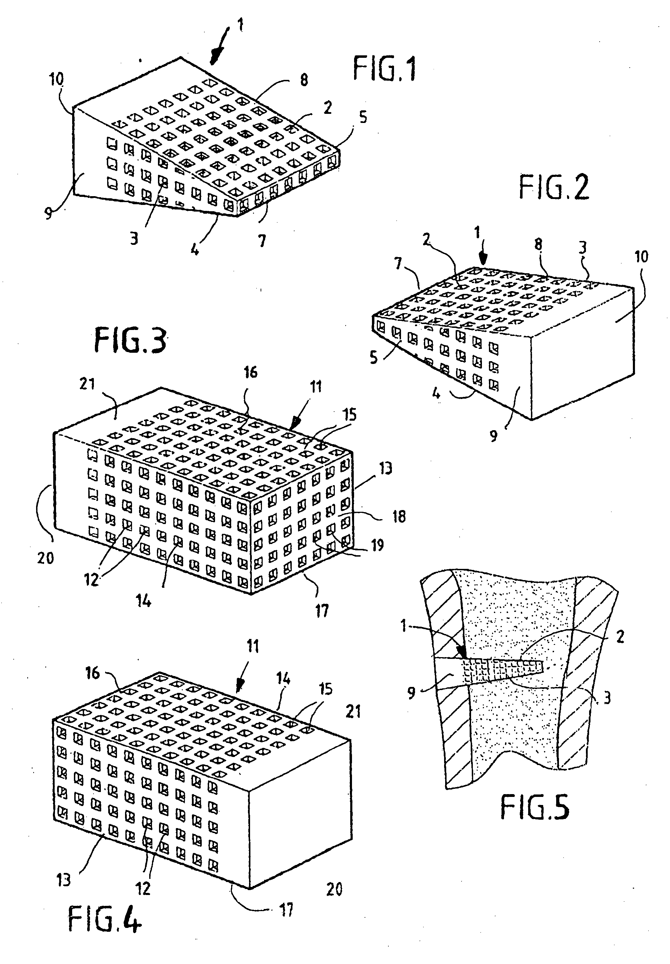

Les matériaux doivent présenter différentes qualités en dehors de leur biocompatibilité. Ils doivent être suffisamment poreux pour permettre la colonisation du tissu spongieux et en même temps suffisamment résistants pour ne pas risquer de s'affaisser sous le poids du corps au niveau du périoste. Au document WO 99/52473 il est décrit une cale tibiale en un matériau résorbable constituée d'un corps creux, notamment au niveau de sa partie proximale. La forme en deux parties est basée sur ce document. L'un des buts de l'invention est de réaliser une cale qui réponde à ces différents critères, et est atteint par une cale telle que définie à la revendication 1. Des perfectionnement sont définis aux sous revendications. L'invention va maintenant être décrite avec plus de détails en se référant à des modes de réalisation particuliers donnés à titre d'exemple seulement et représentés aux dessins annexés.
Figure 1 et figure 2 sont des vues en perspective d'une cale selon l'invention.
Figure 3 et figure 4 sont des vues en perspective d'une variante de réalisation.
Figure 5 montre schématiquement la mise en place de la cale lors d'une ostéotomie.
La cale représentée aux figures 1 et 2 est réalisée en céramique, notamment en phosphate de calcium, par stéréolithographie et affecte la forme d'un tronc de pyramide à base rectangulaire. La cale 1, cunéiforme, comporte une partie proximale 9 pleine et une partie distale qui comporte, sur ses faces latérales 2, 3, 4 et 5 et sur sa petite base 7, de très fins canaux 8. Les canaux de la face 2 s'étendent en direction de la face 4, ceux de la face 3 en direction de la face 5 et ceux de la petite base en direction de la grande base 10. Le volume de la partie proximale pleine est adjacent à la grande base 10, celle-ci ayant une surface continue, c'est-à-dire qui n'est pas percée de canaux. La partie proximale est délimitée, du côté de la partie distale percée, par une face interne.

Une telle cale est plus particulièrement destinée, à l'ostéotomie soit d'un genu valgum, soit d'un genu varum, opération dans laquelle, après avoir pratiqué une fente dans le tibia, au voisinage de l'épiphyse, on insère la cale de manière que la partie percée des canaux vienne se situer dans le tissu spongieux et soit colonisée par celui-ci, tandis que la partie 9 distale pleine est disposée au droit du périoste.

Bien entendu, l'opération est stabilisée par l'addition médiale d'une plaque métaphysaire.

Aux figures 3 et 4, on a représenté une variante de réalisation dans laquelle la cale 11 est constituée par un bloc parallélépipédique percé de canaux 12 s'étendant depuis une face 13 vers la face 14, de canaux 15 s'étendant depuis une face 16 vers la face 17 et de canaux 19 s'étendant à partir de la face 18 mais qui ne s'étendent pas jusqu'à la face opposée 20 afin qu'une partie pleine 21 soit ménagée au voisinage de la face 20.

La cale 11 s'utilise de la même façon que la cale 1, la partie pleine étant disposée au droit du périoste.

Bien entendu, l'invention n'est pas limitée aux modes de réalisation qui viennent d'être décrits et représentés. On pourra y apporter de nombreuses modifications de détail sans sortir pour cela du cadre de l'invention.

## Revendications

1. Cale pour ostéotomie tibiale ou fémorale, constituée d'un corps (1) en céramique ayant une partie distale percée de canaux (12) et une partie proximale destinée à être disposée au niveau de l'os cortical, **caractérisée en ce que** la partie proximale est pleine.

2. Cale suivant la revendication 1, **caractérisée en ce qu'**elle est cunéiforme.

3. Cale selon la revendication 1 ou 2, **caractérisée en ce que** la cale est réalisée par stéréolithographie.

4. Cale selon la revendication 1, 2 ou 3, **caractérisée en ce que** la cale affecte la forme d'un tronc de pyramide ayant une base carrée ou rectangulaire.

5. Cale suivant l'une des revendications précédentes, **caractérisée en ce que** la partie proximale définit un volume (9) plein délimité par une face (10 ; 20) proximale, une ou plusieurs faces latérales et une face interne, la face interne réalisant l'interface entre le volume (9) plein et la partie distale.

6. Cale suivant l'une des revendications précédentes, **caractérisée en ce que** l'extension de la partie proximale correspond sensiblement à l'épaisseur d'un os cortical de tibia ou de fémur.

## Patentansprüche

1. Keil für Osteotomie des Schienbeins oder des Oberschenkelknochens, gebildet aus einem Körper (1) aus Keramik, der Folgendes umfasst: einen von Kanälen (12) durchzogenen distalen Teil und einen proximalen Teil, der dazu bestimmt ist, im Bereich der Kortikalis angeordnet zu werden, **dadurch gekennzeichnet, dass** der proximale Teil voll ist.

2. Keil nach Anspruch 1, **dadurch gekennzeichnet, dass** er keilförmig ist.

3. Keil nach Anspruch 1 oder 2, **dadurch gekennzeichnet, dass** der Keil durch Stereolithografie gebildet wird.

4. Keil nach Anspruch 1, 2 oder 3, **dadurch gekennzeichnet, dass** der Keil die Form eines Pyramidenstumpfes mit quadratischer oder rechteckiger Basis aufweist.

5. Keil nach einem der vorhergehenden Ansprüche, **dadurch gekennzeichnet, dass** der proximale Teil ein volles Volumen (9) definiert, das durch eine proximale Fläche (10; 20), ein oder mehrere Seitenflächen und eine Innenfläche begrenzt wird, wobei die Innenfläche die Grenzfläche zwischen dem vollen Volumen (9) und dem distalen Teil bildet.

6. Keil nach einem der vorhergehenden Ansprüche, **dadurch gekennzeichnet, dass** die Ausdehnung des proximalen Teils im Wesentlichen der Dicke einer Kortikalis des Schienbeins oder des Oberschenkelknochens entspricht.

## Claims

1. Wedge for femoral or tibial osteotomy, made of a body (1) in ceramic material, having a distal part through which canals (12) are perforated and a proximal part to be disposed at the cortical bone, **characterized in that** said proximal part is solid.

2. Wedge of claim 1, **characterized in that** said wedge is of cuneiform shape.

3. Wedge of claim 1 or 2, **characterized in that** said wedge is made by stereolithography.

4. Wedge of claim 1, 2 or 3, **characterized in that** said wedge has the shape of a pyramidal trunk having a square or rectangular base.

5. Wedge as defined in one of the previous claims, **characterized in that** said proximal part defines a solid volume (9) delimitated by a proximal face (10 ; 20), one or more lateral face and an internal face, said internal face making the interface between said solid volume (9) and said distal part.

6. Wedge as defined in one of the previous claims, **characterized in that** the extension of the proximal part substantially corresponds to the thickness of a cortical bone of the tibia or femur.
